# EUROPEAN PATENT SPECIFICATION

(11) **EP 0 604 563 B1**
(45) Date of publication and mention of the grant of the patent: **17.03.1999**
(21) Application number: 92920634.0
(22) Date of filing: 18.09.1992
(51) Int. Cl.: A61F 7/12, A61M 29/02, A61B 17/38

(54) **DEVICE FOR HYPERTHERMIA TREATMENT**
VORRICHTUNG ZUR BEHANDLUNG MITTELS HYPERTHERMIE
DISPOSITIF POUR LE TRAITEMENT PAR L'HYPERTHERMIE

(30) Priority: 20.09.1991 SE 9102728; 16.04.1992 SE 9201224
(43) Date of publication of application: 06.07.1994
(73) Proprietor: WALLSTEN, Hans Ivar, CH-1141 Denens (CH)
(72) Inventor: WALLSTEN, Hans Ivar, CH-1141 Denens (CH)
(74) Representative: Burman, Tore
(86) International application number: SE9200645
(87) International publication number: WO9305737

(56) References cited:
- EP-A- 0 253 677
- EP-A- 0 333 381
- WO-A-90/02525
- DE-A- 3 725 691
- DE-C- 3 516 830
- US-A- 4 160 455

## Description

The present invention relates to an apparatus or a device for carrying out so called hyperthermia in a body cavity or body canal by heat generation.

Heat generated through different energy forms is used inter alia in the field of hyperthermia, which involves the treatment of tissues of different kinds with the supply of heat. Hyperthermia is particularly used for the treatment of malign tumors, whereby one makes use of the fact that certain cancer cells are destroyed at a certain temperature, for example about 42°C, whereas surrounding healthy cells remain unaffected at this temperature. Hyperthermia is also used in the treatment of certain benign conditions of disorder, for example prostate hyperplasia (BPH), where heating of the swollen prostate rectally or urethrally to a temperature range of about 44-48°C gives a certain positive effect, probably depending on the fact that the swelling is reduced.

Heat is utilized also medicinally in that laser beams or instruments heated by radiofrequency are introduced at certain surgical operations, the tissue being burned at the same time as the blood of surrounding vessels is brought to coagulate. In this manner also certain organ parts showing large flow of blood (perfusion) are treated.

A pronounced problem in hyperthermia resides in measuring and controlling the temperature in the tissue to be treated so as not to destroy surrounding healthy tissue. This is particularly the case with deeply located tissues or organs. Irrespective of the manner at which heat is treated, such as by heat conduction or through ultrasound, radiofrequent energy, microwaves, differences in the structure and flow of blood of the tissue contributes to difficulties with regard to maintaining a certain temperature or obtaining a certain depth of treatment.

The choice of method of treatment is affected by these factors and it is thus for example not possible to establish a desired temperature level by means of heat conduction from a heat source in view of the fact that the temperature drop inwardly through the tissue becomes too large depending on the cooling effect of the blood. The temperature distribution in different parts of the treated organ also often becomes uneven in view of variations in the flow of blood. Also in the treatment of more deeply penetrating methods, for example using radio frequency techniques in the frequency range up to 300 MHz or microwave techniques in the frequency range 300-2450 MHz, varying blood perfusion raises problems, partly in view of the fact that the temperature gradient inwardly in the tissue varies, partly through the fact that the temperature will vary within different parts of tissue.

As indicated above also temperature measurement constitutes a problem. It is often impossible to measure the temperature at a sufficient number of locations in the tissue volume to be treated, a condition which is aggravated by varying perfusion which results in a need of an even larger number of measuring locations.

It has been suggested in the treatment of certain tumors to introduce by operation metal needles at a certain distance from each other, said needles being made of a ferromagnetic material are capable of heating wirelessly through magnetic induction. This technique means that the needles are left for a number of treatments and in each treatment the patient is placed so that an exterior induction coil surrounds the part of the body containing the tumor involved. This coil generates a magnetic field, the intensity and strength of which affects the heat released from the needle by heat conduction. By selecting a metallic material among certain alloys having such magnetic properties as to have a so called Curie point corresponding to the desired temperature no particular thermoelements for measuring the temperature are required. Such needles of so called ferromagnetic alloys operate as if had they a built-in thermostate and also generate during the heating phase less heat the closer one comes to the desired temperature (the Curie point). The absence of equipment for temperature measurement and temperature control is, of course, a great advantage, but the drawback of this particular technique is the fact that the needles have to be capable of producing sufficient quantities of energy so as to reach the desired treatment temperature. On the other hand the needles must not be too thick or must not flex too easily, and therefore the technique is not suitable for application on organ parts where the blood perfusion is high or uneven. Another drawback associated with the technique is that it cannot be applied to the treatment of difficultly accessible tumors or intra-cavitarily.

Heat treatment has also been applied to excessive uterus hemorrhage (menorrhagia), which disorder shows very strong bleeding as a symptom. The most common measure adhered to at this disorder is uterus extraction, which is a traumatically very large surgical operation associated with certain risks and which also can result in conditions of psychical stress. Only in the United States about 0.5 million of hysterectomies are made, the majority thereof because of menorrhagia.

For a long time one has tried to develop methods for the suitable treatment of the disorder, inter alia by destroying the uterus mucosa (endometrium) by heating, for example using a laser, but this is time consuming and requires pronounced caution and skill. Attempts using microwave heating have not been very successful, since the uterus cavity is flattened and triangular shaped and the distribution of the microwaves is such as to result in a very uneven treatment. The large flow of blood in the endometrium surrounding the myometrium also results in large temperature drops in view of the cooling effect of the blood, resulting in uneven heating. There are also difficulties to protect the cervix and the vagina from burns. Moreover, experience shows that perforation of the uterus wall can take place with the treatment using a laser.

Prior art involving techniques of some relation to the present invention is disclosed in international application WO90/02525 and EP A2 0 333 381. This international application relates to an intrauterine cauterizing apparatus and method involving an applicator comprising a distendable bladder connected to a catheter, said bladder being expanded by introducing a non-toxic fluid under pressure. Said fluid is then heated to cause necrosis of the tissue lining of a body cavity. This apparatus operates without circulation of the fluid and uses a conventional heating element supplied with electric power.

The above European patent application relates to a thermal seed for the treatment for tumors, said seed involving a ferromagnetic material which is excited by a coil generating a magnetic field to produce heating of the core. This technology only involves the heating of a core of ferromagnetic material for direct influence of surrounding tissue without resorting to the use of a fluid medium for the transfer of heat to the tumor tissue.

US patent 4,160,455 discloses a device according to the first part of claim 1.

US patent 4,860,744 describes a system and method for providing precisely controlled heating of a small region of body tissue such as to effectuate removal of tumors and deposits. This system relies on thermoelectric and resistive heating and thermoelectric control of a heated probe tip. However, the system does not use a heated balloon with internal circulation and does not suggest the use of a heating element of the self-regulating type.

DE A 37 25 691 relates to a heating device for the destruction of bodily tissue subject to disorder and relies on a balloon catheter containing a heating medium containing a heating element exemplified by a PTC-type of element. The element is of a one-part type and the device is not arranged for internal circulation of the heating medium.

Further examples illustrating prior art concerning the treatment of internal disorders while supplying heat and optionally supplying pressure are found in US patents No. 4 160 455, 4 773 899, 4 799 479, 4 709 698 and 4 949 718, and the EP-application, publication No. 0 370 890 A1.

The present invention has for its purpose to provide new techniques for carrying out hyperthermia, whereby the drawbacks of the prior art are eliminated or at least substantially reduced.

One particular object of the present invention is thus to provide a device for carrying out such so called hyperthermia in a body cavity or duct in a manner which is satisfactory from a safety point of view. For these and other objects the device according to the invention comprises the features as defined in the claims

As will be explained later in this disclosure heat-releasing elements of the inherently self-regulating or self-controlling type are exemplified by materials of the PTC-type.

The PTC-material is a semiconductor and has a constant resistance up to a specific temperature, the so called Curie Point (CP) or trip point (TP), which is predetermined. At the trip point, the resistance increases by 100% but over this temperature the resistance increases drastically, for example 20-30% per °C.

When the material is connected to an electric source of a certain voltage it will emit energy in form of heat from its surfaces in such a way that the larger the surface the greater the emission will be. Because of the temperature-depending resistance, it is self-regulating with regard to heat emission at temperatures in the range of the TP, e.g. the material is capable of maintaining steady state conditions even if variations in power requirement occur at a certain temperature level.

According to the invention, the centrally located heating element consists of a self-regulating material of a compact design enabling access to the treatment site but at the same time providing sufficient heat-emitting surfaces to be capable to emit sufficient energy at a certain temperature level even at variations in power.

The problem of creating sufficient power output while avoiding self-inhibition associated with a compact design of the heating element has been solved in accordance with the invention by arranging the compact heat releasing self-regulating material in a surrounding elongate housing or by designing the material itself as an elongate housing with an inlet and an outlet, the heat-transmitting medium being forced through and around the material by an efficient internal circulation. Inspite of restriction in size the material can provide for sufficient power output at temperatures in the range of TP or CP, thus resulting in successful treatment under self-regulating conditions. This also means that the heat-emitting element above the predetermined temperature range of CP operates in such a manner that the energy-generation automatically will be significantly reduced and self-inhibition will occur so that overheating thereby cannot take place under any circumstances.

For the treatment of disorders of the uterus the material of the heat-releasing element may have a trip point or Curie point lying within the range about 60°C to about 90°C, particularly about 70°C to about 80°C. On the other hand, for the treatment of prostatic disorders said ranges may be from about 45°C to about 65°C, particularly about 48°C to about 56°C.

In accordance with the invention the device contains in said elongate housing two or several, axial and parallelly arranged part-elements of PTC-type having substantially parallel surfaces. Said part-elements are suitably placed spaced from each other and from the surrounding housing wall to form canals or passages between and about the part-elements to form sufficient surface and to provide for effective and even heat-emission to the surrounding flowing heat-transmitting medium.

In order to obtain a sufficient power output the PTC-material is preferably arranged with a packing degree within said housing within the range about 35 to about 70% by volume, i.e. corresponding to a void volume of about 65 to about 30%, preferably about 45 to 65% by volume, i.e. corresponding to a void volume of about 55 to about 35%.

In such preferred embodiment the thickness of the part-elements is suitably at most about 1.5 mm and they may have a power capacity at steady state of at least about 1-1.5 W/cm² of exposed element surface at a voltage of about 24 V. In this disclosure the expression "exposed element surface" refers to the total surface of the heating element exposed to the heat-transmitting medium. Due to the lower electrical resistance at temperatures below the self-regulating temperature range the emitted power will be much higher, such as 3 times higher than at steady state, which is of a great advantage since it significantly reduces the start-up time.

For use in narrow passages in a living body said housing preferably has an outer diameter of at most about 7-8 mm, and at such dimension its capacity of heat-generation at steady state shall correspond to a release of heat of about 3 W/cm axial length or extension of the housing at the treating temperature used and at a voltage of about 24 V.

It is preferred that said means for internal circulation has a capacity at steady state conditions of replacing the void volume of the housing of said medium at least about 30 times per minute, and preferably at least about 60 times per minute.

According to a particularly preferred embodiment of the invention the device may contain an inlet for the medium located in the housing on the opposite side of element relative to said outlet, and means for imparting flow to the medium in an internal circuit from the space between housing and the surrounding flexible enclosure, through housing via said inlet and outlet, for absorbing heat and through the space between housing and enclosure for the release of heat through the enclosure wall.

In such embodiment of the invention the device may be further characterized by at least one first back valve arranged in association with the inlet, said back valve allowing flow into the interior of the housing, and the flow resistance of which in an open position is lower than the flow resistance through the housing, said means for the flow of the medium in an internal circuit being arranged to provide a reciprocating movement of a small quantity of the pressurized quantity of medium enclosed in the inlet canal of the device after expansion of the enclosure, whereby the inlet is closed and the outlet is open, whereby the circulation of the medium in a circuit is provided.

According to yet another embodiment of the invention the device is further characterized by at least one first back valve arranged in association with the outlet allowing flow out of the interior of the housing, the flow resistance of the inlet being higher than the flow resistance through the housing, said means for the flow of the medium in an internal circuit being arranged to provide a reciprocating movement of a small quantity of the pressurized medium enclosed in the inlet canal of the device after expansion of the enclosure, whereby the inlet is open and the outlet is closed, thereby providing circulation of the medium in a closed circuit.

According to an alternative embodiment of the invention the device is further characterized by an inlet for the medium arranged in the housing on the other side of the element relative to said outlet and by a partition placed between said inlet and the element or between said element and the outlet, respectively, forming a chamber and which is provided with an axial passage containing a second back valve, which is oppositely placed relative to the first back valve placed in the inlet, the means for the flow of the medium in an internal circuit being arranged to provide a reciprocating movement of a small quantity of the pressurized medium enclosed in the inlet canal of the device after expansion of the enclosure, whereby the inlet is closed and the outlet is open or the inlet is open and the outlet is closed, respectively, thereby providing circulation of the medium in a closed circuit.

It is preferred in the device according to the invention to provide two oppositely placed back valves in connection with the inlet and the outlet. Said partition is suitably placed between the inlet and the element.

The back valves used in association with the present invention can be of any conventional type. As examples there may be mentioned flap valves, ball valves, disc valves, etc. Flap valves are preferred in view of their simple design.

Said means for providing flow of the medium may, in accordance with a preferred embodiment of the invention, be arranged to provide for a volumetric flow of the medium. Such means may comprise a reciprocating piston.

According to an alternative embodiment of the device according to the invention said means for the flow of the medium in an internal circuit include a propeller or an axial pump wheel placed between the radial inlet and outlet.

As an alternative, to provide for circulation of the medium the device may contain means comprising a reciprocating piston provided with one or several axially directed apertures and corresponding back valves.

Also as indicated above, the device according to the invention is particularly suited for the treatment of disorders in uterus, for example menorrhagia. Alternatively, the device according to the invention can be used for the treatment of prostatic disorders.

In connection with the use of a self-regulating system containing a semiconductor material of PTC-type is particularly preferred from a safety point of view to feed the semiconductor material using a low-voltage current, for example having a voltage of at most about 50V and particularly at most about 30V. Using available semiconductor materials of PTC-type even lower voltages can be used, such as down to 20 to 25V, enabling use of rechargeable batteries. This excludes the risk for electric shocks.

The controlled pressure used in combination with heat supply is suitably at least a pressure lying within the range between diastolic pressure and systolic pressure.

The invention will in the following be further described by exemplifying embodiments which, however, must not be considered to restrict the scope of protection as defined in the appended claims. These embodiments are described in connection with the appended drawings, wherein:
Fig. 1 illustrates diagramatically an embodiment of the device according to the present invention working with pulsating pressure;
Fig. 2 shows in enlargement a section through the interior central distal part of the device shown in Fig. 1;
Fig. 3 shows a diagram on electric resistance as a function of temperature for a PTC-material used;
Fig. 4 shows yet another embodiment of the device according to the invention, more particularly its distal part with alternative means for providing an internal flow circuit;
Fig. 5 shows another embodiment of the device according to the invention, i.e. the distal part thereof, suitable for the treatment of for example prostate hyperplasia;
Fig. 6 shows in a cross-section different embodiments of heat-emitting elements containing a PTC-material; and
Fig. 7 shows a diagram on temperature/power as a function of time of treatment in connection with an experiment made.

According to the invention a self-regulating system is used, i.e. a system wherein the energy generation falls rapidly at a certain predetermined temperature in dependence on properties of the heat-emitting material. Practical examples are presented below.

According to the present invention the heat-emitting element is constituted by a semiconductor material of a certain character, a thermistor of PTC-type (Positive Temperature Coefficient). This material consisting of a ceramic material usually originating from BaTiO₃ has the property that the electric resistance is dependent on the temperature in such a manner that the resistance is constant and low up to a certain temperature, the so called Curie temperature or trip point, whereas the resistance increases rapidly if the temperature exceeds the Curie or trip point temperature. Such semiconductor materials are presently sold by for example Siemens and Philips and are used practically for heating, for example in ironing devices for travelling, rear view mirrors etc. As mentioned earlier the self-regulating material according to the best mode of the invention is enclosed in an elongate housing or constitutes itself the elongate housing which diameter shall not exceed 7-8 mm and preferable not 5 to allow the housing which constitutes the distal end of the catheter to pass through narrow passages such as the cervix canal or the urethra. There is also a limitation in length of the energy emitting part of the housing. When used for the treatment or uterus it is preferred that the length is not longer than the depth of the uterus which normally is between 30 to 80 mm. The reason is that it is not desirable that the cervix canal is heated. In case of the treatment of prostatic disorders there is also a limitation in length determined by the distance between the sphincter and the bladder.

Consequently the volume of the heat emitting part of a housing with a length of 3 cm shall not have a volume of more than about 1.5 cm³, preferably not more than 0.6 cm³ equal to 0.2-0.5 cm³/cm length. The corresponding heat emitting surfaces are about 4.5 to 7.5 cm² - these numbers only given as examples.

Trials have shown that high energy emissions are necessary under treatment to compensate for the heat losses through the uterus will, which has a surface of about 14-40 cm², e.g. 3 to 10 times larger than that of the heating element.

Trials have also shown that there is a need of power of about 8-40 W to compensate the heat losses in the treatment of uterus, i.e. at the temperature where self-regulation occurs, depending on the specific circumstances, such as the penetration deep of heat, blood perfusion, size of the uterus cavity. Even if a large and deep uterus allows a longer heat emitting part there is a minimal need of about 3 W per cm length for a device according to the invention. For the treatment of a deeper and larger uterus a longer emitting part however can compensate for the larger need of power.

The PTC material can be produced in many different shapes. For the use as heating elements for some of the embodiments according to the invention it is preferred to use elements in flat or tubular forms. A flat PTC material has normally a thickness of about 1 mm or more. The two sides are covered with a layer of electrical conductive material. When the two sides are connected to an electric source heat is developed in the material depending on the resistance of the material. The amount of heat at a certain temperature below the TP is depending of different factors, such as the material characteristics, the voltage applied but also the size of the surface. One advantage is that considerable amounts of heat can be emitted from a certain surface area.

To be able to fulfill the requirement to have a minimum emission capacity of 3 W per length cm in a housing of 0.2-0.5 cm³ per cm length as mentioned as examples above the material however has to be enclosed and packed in the housing in a very dense way.

Experiments have shown that to achieve sufficient power emission capacity the packing degree of PTC material will be between about 35-70% of the total volume of the housing. As mentioned earlier such high density causes self-inhibition even if there are arranged passages in between elements of the PTC material, unless an efficient circulation of a heat-absorbing medium is provided.

These passages are filled with a heat-absorbing medium in form of a liquid which is forced to pass through the passages in the housing. By an effective circulation of the liquid the emitted heat is taken up by the liquid at its passage through the passages in the housing and brought out through the outlet into the outer space between the housing and the expanded enclosure to compensate for the heat losses through the enclosure to the surrounding endometrium. It is preferred that the circulation means consist of a volumetric pump to provide for an efficient circulation of liquid in spite of the resistance imparted by the narrow passages. Under the condition that the flow of circulating liquid, e.g. water, is sufficient, the amount of emitted energy in the form of heat from the element will at steady state conditions compensate for the heat transfer to the endometrium.

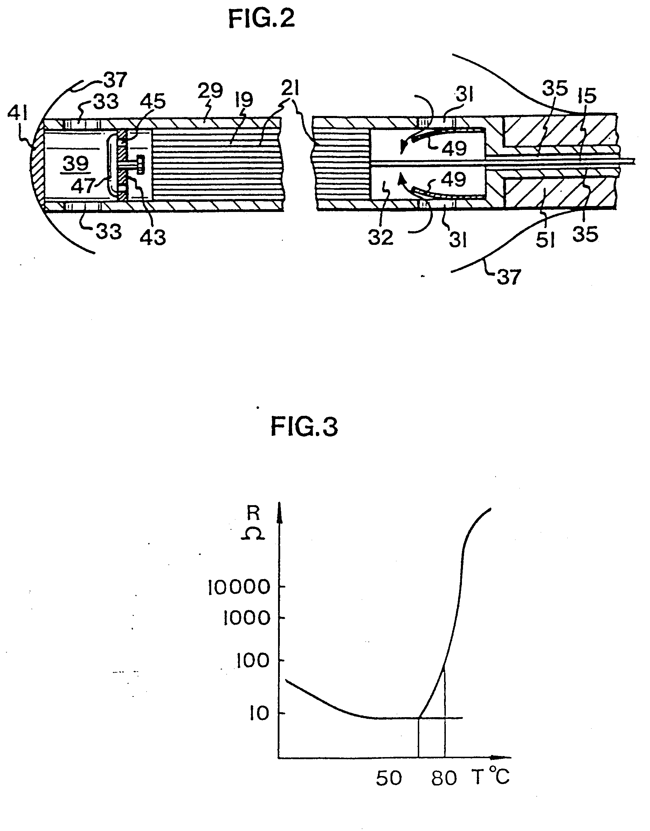

The temperature-resistance curve for a suitable PTC-material is shown in Fig. 3. A PTC-material having a suitable Curie point and a relation between resistance and temperature in principal similar to that of Fig. 3 becomes self-regulating as regards the generated power at the temperature desired for the treatment. Thus, for example a suitable material having a Curie or trip point of about 70°C (see Fig. 3) and is supplied with 20 volts can generate up to about 20 W/cm length of element during the heating-up phase and about 7 W/cm length at a temperature of about 65-70°C. However, if the temperature exceeds 80°C the generated energy falls drastically, and self-inhibition occurs after a few degrees increase. In hyperthermia according to the invention it is possible to maintain uterus surface at a desired treatment temperature, for example about 70°C. One condition for this is that the corresponding energy requirement to cover the heat losses out into the uterus wall can be provided. In view of the properties of the PTC-material and as is clear from Fig. 3 the heat release will automatically adjust so as to correspond to the power needed at a specific moment. If the cooling from the uterus wall for some reason suddenly increases the surface temperature drops somewhat resulting in an increased heat release from the PTC-material. On the other hand the temperature cannot exceed a certain value if for example the power requirement becomes very small. In other words, the material never becomes hotter than this highest temperature, for example 80°C, which makes this system totally safe and cannot result in overheating.

There are other advantages of the said PTC-material as a heat-emitting medium. Since the system is self-regulating with regard to energy and temperature no more or less complicated thermometry will be needed, nor a more or less sophisticated control system. Furthermore, the device is suitable for mass production and to a low cost therefore making it suitable as a disposable.

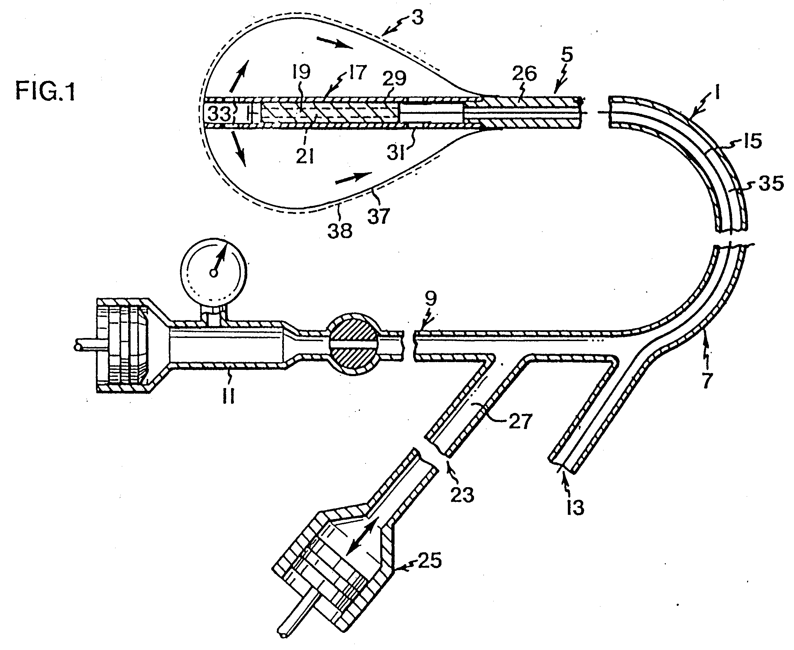

The device according to Fig. 1 includes a heat applicator 1 in the form of a catheter of a disposable type, the distal part 3 of which is intended to be inserted into the body cavity, for example the uterus cavity, that shall be heat treated. The applicator has an intermediate part 5 for facilitating insertion of the distal part 3 and for fixation of the position of part 3 in the cavity. The proximal part 7 of the applicator has connecting means 9 for the supply of a compressed medium, for example a liquid from an apparatus 11, for the generation and measurement of pressure via an inlet canal 35 associated with the applicator. Furthermore, the applicator has a connecting means 13 to an electric energy source, not shown, so that an electric current via a cable device 15 can be supplied to a central body 17 containing inter alia a heat-emitting element 19 of a self-regulating type, such as PTC-type, and canals or passages 21 arranged therein or thereabout. Furthermore, the applicator has connecting means 23 to an apparatus 25 for the generation of oscillating pressure impacts to the inlet canal 35 of the applicator after it has been filled with pressurized liquid from apparatus 11. The central body 17 is surrounded by a thin flexible and elastic enclosure or balloon 37 which is brought to expand when the pressure medium is supplied to the interior of the enclosure through canal 35.

The central body 17 with the heat-emitting element 19 has an elongate shape and has a housing 29 surrounding element 19, the housing being provided with apertures 31 in its proximal part and apertures 33 in its distal part.

Fig. 2 shows in detail of the embodiment according to Fig. 1 a system for enabling effective heat transport and heat transfer from the heat-emitting element 19 to the uterus mucosa (endometrium). Apertures 31 in the proximal part of the tubular central body act as radially acting inlets for a pressurized liquid to a valve housing 32 communicating with the inlet canal 35, the space between central body 17 and enclosure 29, as well as with canals 21 arranged about and through the heat-emitting element 19 in the middle part of the central body. Apertures 33 arranged in the distal part of the central body act as outlets for the pressurized liquid from a second valve housing 39 communicating with canals 21 and the space between the central body 17 and the elastic enclosure 37, said enclosure being liquid-tight sealed around the proximal part of the central body at 51 and being attached to the nose member 41 of the central body. In valve housing 32 back valves 49 are arranged so as to close apertures or openings 31 at overpressure in the valve housing 32 and opening at a sub-pressure relative to the liquid pressure in the space between central body 17 and the elastic enclosure 37. A partition 43 having openings 45 is arranged in valve housing 39. A disc valve 47 is moveable in axial direction and arranged so as to close openings 45 at over-pressure in valve housing 39 relative to the liquid pressure in canals 21 in the heat-emitting element of the central body or opening at sub-pressure in valve housing 45, respectively.

During heat treatment of for example uterus using the device according to Fig. 1 means 9, 13, 23 are connected to the respective apparatuses for the supply of pressurized liquid, electric power and for the generation of oscillating pressure impact. Then the distal part 3 of the applicator is inserted through cervix into the uterus cavity up to the bottom of the cavity. The length of central body 17 corresponds approximately to the length of the uterus cavity, i.e. normally 4-5 cm. Pressurized liquid is then supplied to the applicator through apparatus 11 via the inlet canal 35, back valve 49 closing apertures 31 and disc valve 47 being open, whereby the thin flexible and elastic enclosure 37 in the form of a balloon expands so as to conform to the uterus cavity and bring the enclosure to adapt to the contoure of the cavity and to completely engage under pressure the endometrium of the cavity, i.e. in the position shown in Fig. 1. By means of the apparatus 11 for the generation of pressure the liquid pressure is then maintained at a level suitable for the treatment, which shall be described later.

Then apparatus 25 having an oscillating volumetric pump, for example a piston pump, is started. At every positive pressure shock a certain volume of liquid is brought to move forward through the inlet canal 35 of the catheter body, which has for an effect that simultaneously a corresponding quantity of pressurized liquid is forced through canals 21 in the central body 17 in view of the fact that valves 49 are closed at the same time as valve 47 is opened and a corresponding quantity of pressurized liquid will be pushed out into the space inside the balloon enclosure 37. In view of the pressure increase balloon enclosure 37 and the uterus muscle surrounding same will be somewhat extended, which has been indicated with dashed lines 38 in Fig. 1.

At every subsequent withdrawal in the movement of the piston pump a corresponding liquid quantity will be sucked back, valves 49 taking the open position shown in Fig. 2 and valve 47 its closed position. The enclosure takes at the same time its original position according to the full line 37 in Fig. 1.

It is appreciated that under the influence of the oscillating pressure shocks and the described valve system a powerful and effective circulation in an internal flow circuit of liquid at the pressure given by pressure apparatus 11 will be provided through canals 21 of the central body 17 and out into the space within the balloon enclosure and back to the central body without hot liquid passing the inlet canal 35 of the catheter to connecting means 23. Circulation thus takes place only in the distal part 3, whereas the inlet canal 35 during the circulation only serves as a communication conduit for transmitting in a hydraulic manner the oscillating pressure and liquid movement provided by the pump apparatus 25. Therefore, the canal can have a relatively small diameter but should be flexible and should not expand significantly in a radial direction at the pressure increase in view of the strokes of the pump. In view of the small diameter of canal 35 space is admitted for a heat insulation 26 surrounding the intermediate part 5 of the catheter in order to protect the delicate cervix during treatment. In view of the circulation system described an effective circulation of liquid is made possible also through canals 21 which in certain embodiments offer a great resistance, which shall be described later. Another advantage is the fact that the quantity of circulating liquid is determined by length of stroke and frequency of the pump and that thus the quantity can be controlled and varied in a simple manner and is independent on different changes in circulation resistance, as for example different sizes and shapes of the cavity.

A preferred liquid for use as a heat-emitting medium is water, either as such or as an isotonic salt solution. In the embodiment described and for the treatment of menorrhagia, it is preferred that the circulation through the housing is at least about 5 ml/min and per cm axial length of the element after reaching steady state conditions, i.e. the temperature of treatment.

In the circulation device described with reference to Figs. 1 and 2 the circulation takes place by the liquid first being forced into the proximal part of the central body 17 and will then leave the distal part of the central body. It is understood that a reversed direction of circulation can be obtained if for example back valves 49 are arranged at the outside of openings 31 and the disc valve 47 is mounted reversely in the partition 43 so as to open for apertures 45 at an overpressure in valve housing 39.

When selecting a system for heat generation the safety aspect must, however, be decisive. Thus, it is important that the risk for electro shocks in view of defect parts is excluded.

Another requirement from a safety point of view is the fact that the temperature necessary for the treatment of the tissue can be maintained during the course of treatment and that this temperature will not be exceeded. There is a further requirement that the different parts that constitute the central body must not be overheated. Otherwise there is a risk that for example housing 29, if this comes into contact with the enclosure 37 the latter may be punctured. Furthermore, the patient may be subjected to burns. These requirements for safety are fulfilled in the different embodiments within the scope of the invention.

It has been found important for the result of the treatment that the temperature necessary for the treatment can be maintained over the whole of the surface to be treated and that the necessary energy requirement for maintaining the temperature can be accomplished and that sudden changes in energy requirement in view of for example variations in blood pressure of the patient can be met quickly by a corresponding change in the power output.

These claims can he met by different embodiments within the concept of this invention. In view of the fact that the heat-emitting element 19 emits its heat to the flowing liquid said liquid will in turn release its heat over the whole of the surface of the uterus mucosa by heat conduction through the thin enclosure 37. In this manner large quantities of heat can be supplied over the whole of the uterus mucosa.

By the device shown in Fig. 1 the enclosure, as mentioned, can be brought by influence of pressure to conform to the contours of the expanded cavity and in this manner enable simultaneous treatment of the whole surface. With the device according to the invention there is also obtained another important advantage, since one by suitable choice of pressure through pressure effect on the mucosa and the underlying tissues can restrict the flow of blood in veins and arteries and in this manner reduce or completely prevent the cooling effect of the blood on the heat supplied from the central body by heat conduction. It has been surprisingly found, that the heat conductivity increases manyfold in a tissue flown through by blood and subjected to pressure as compared to the same tissue without pressure. It has also been found that the heat treatment becomes more even both laterally and depthwise.

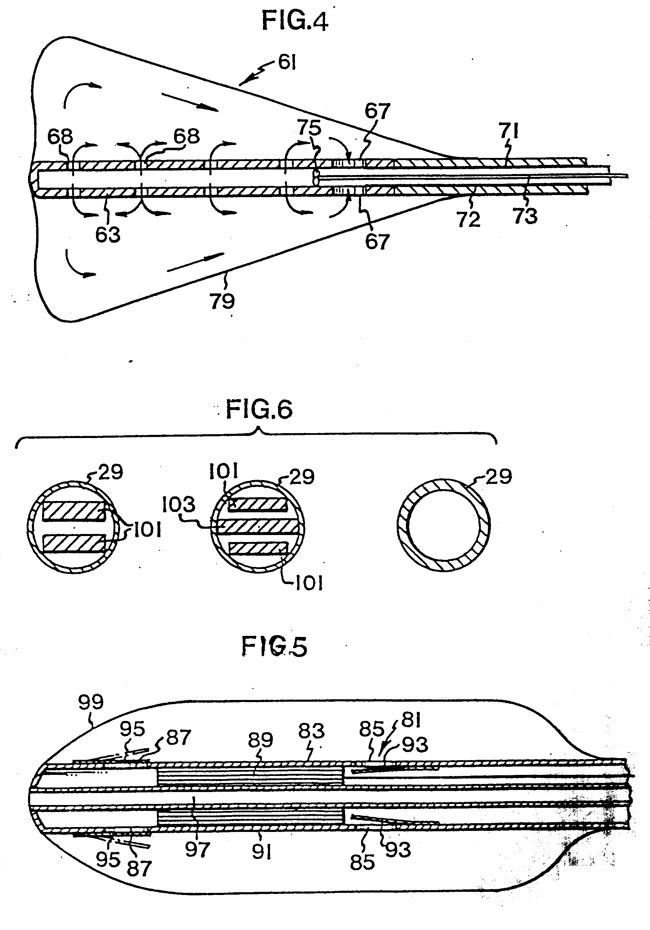

Fig. 4 shows in detail the distal part 61 of a catheter. The catheter has a design similar to that of Fig. 1, i.e. it has connecting means for a pressure source for the supply of liquid under pressure and connecting means to a cable for the supply of electric energy (not shown) for the generation of heat. The connecting means described in Fig. 1 for oscillating pressure is, however, not present. The necessary circulation is, instead, performed in a mechanical manner by means of a rotating flexible shaft 73

Central tube 63 consists of a PTC-material and has inlet apertures 67 and outlet apertures 68 for a flow of liquid distributed along and around the tube. The current leads 71,72 for the element are connected to the inside and the outside, respectively, of the PTC-tube. The shaft 73, which is connected to a driving motor not shown in the figure, imparts fast rotation to a propeller or a pump wheel 75. Through this the energy will be converted to heat, primarily within the cylindrical volume of tube 63. In view of the fast rotation of the pump wheel the liquid will be brought to circulate in a circuit from the inlet apertures 67 through the central tube 63 out through the outlet apertures 68 and via the space between the enclosure 79 and the central tube 63 back to the inlet 67, thus removing heat from the inner surface as well as the outer surface of tube 63.

Since in this case the passage through the central tube does not offer significant resistance it is sufficient with a propeller to obtain sufficient circulation of the liquid.

The choice of pressure in hyperthermia treatment according to the invention is dependent on the disorder to be treated. When treating for example menorraghia high pressures have to be used, since the uterus musculature is yielding at the same time as the mucosa or endometrium and underlying functionalis and basalis layers have a rich flow of blood. By selecting a pressure of at least the diastolic pressure, i.e. about 80 mm Hg, and in certain cases up to the systolic pressure, i.e. about 150 mm Hg, the flow of blood through endometrium can be interrupted and the desired destruction of the basalis layer can be obtained by simultaneous influence of heat, whereby the disorder can be eliminated.

When treating for example benign prostate hyperplasia (BPH) there is suitably used an applicator in the form of a catheter, the distal part of which is shown in Fig. 5. The applicator, the outer diameter of which should not exceed about 7 mm, is inserted into the urethra through penis so that its effective heat-emitting distal part covers the area between the sfincter and the urinary bladder. In the housing 83 of the central body 81 there are proximal inlet apertures 85 and distal outlet apertures 87 arranged. A cable for electric energy is connected to a heat-emitting element 89 with canals 91 for liquid. The heat-emitting element is the previously described PTC-semiconductor. The circulation can suitably take place in accordance with the previously described pumping using pressure shocks. In such a case the in- and outlet apertures are suitably provided with back valves 93 and 95, respectively, according to Fig. 9. A through-going passage 97 is arranged through the center of the applicator for the use of guide-wire or endoscope for positioning.

Alternatively, the correct positioning can be provided by an expandable balloon attached to the distal part of the applicator. In this case the positioning takes place by inserting the applicator so far that part of the distal section enters the urinary bladder. The balloon is then blown up and the applicator is retracted until the expanded balloon prevents further retraction. A suitable treatment temperature is about 45-55°C for a period of 60 min. The selected pressure can advantageously be high, such as within the range about 2-4 atmospheres which not only increases heat penetration but also contributes to enlarge the restriction in a mechanical way. Thus, it has been found possible to treat certain forms of BPH using a heat applicator according to Fig. 5, but where the enclosure 99 is constituted by a flexible polymer material but with limited elasticity. The enclosure, which suitably is preformed to a diameter of about 25-30 mm, is connected to a source of pressure of several atmospheres. The combination of compression of the swollen prostate and heat influence of the tissue around the urethra seems to give a useful effect, such as the formation of a necrosis which is then transformed to connective tissue. This will cause enlarging and strengthening of the urethral duct preventing renewed narrowing of the urethral passage way.

According to an alternative embodiment of the device according to the invention showing great similarities with that shown in Fig. 4 an alternative system for providing the internal circulation is used. Instead of using a propeller or pump wheel in accordance with Fig. 4 such alternative device uses a reciprocating piston which is connected to a shaft that instead of rotation performs a reciprocating movement. Such piston is provided with axially directed apertures and associated back valves, such as flap valves. At the reciprocal motion of the piston internal circulation of liquid will be provided.

Fig. 6 shows in cross-section some preferred embodiments of central elements containing PTC-material in accordance with the invention and the appended Table 1 shows the extraordinary high power outputs that can be obtained from such elements even when the outer diameter of the central tube is small. For comparison an effective length of the element amounting to 30 mm has been chosen, which is a length matching for example an instrument corresponding to that shown in Figs. 1 and 2 for the treatment of for example uterus.

As previously indicated it is desirable for passage of cervix that the outer diameter of the central tube is less than about 7 mm for practically useful performance.

According to Fig. 6, in the PTC-elements of type 1 and of type 2, the central body is designed from a thin cylindrical housing corresponding to housing 29 in Fig. 1. In said housing several axially extending parallel thin plates 101,103 of a PTC-material are arranged. In these examples there has been used as a PTC-material one manufactured by Siemens in the form of thin plates having a thickness of 1.1 mm and having the designation P350 and a Curie point of about 80°C. These plates have metalized planar surfaces to which current leads can be connected, for example by soldering. When a voltage of for example 24 V is supplied to the leads heat will be generated in the interior of the plate and a temperature gradient towards both surfaces of the plate will be obtained, in view of which said surfaces are in contact with circulating liquid in order that the desired power output shall be obtained.

It has now been found that by arranging the plates with axial extension in several layers using a certain space in between the layers within the housing, as shown for example by types 1 and 2 of Fig. 6, it is possible to obtain a very high power density, at the same time as longitudinally extending passages are formed between the surfaces of the plates and the walls of the surrounding housing, said passages offering a certain flow resistance, yet enabling effective flow of liquid. As an example the space in between the plates of type 2 is about 0.5 mm. It is preferred that the circulation in these two cases is provided by a volumetric pumping as previously described in connection with Figs. 1 and 2.

In the embodiment according to type 3 of Fig. 6 the central body is constituted by a tube of PTC-material. The resistance against flow of liquid for removal of heat from the outer and inner surfaces of the tube is in this case low and can, accordingly, be provided using a propeller or pump as described in connection with Fig. 4.

In appended Table 1 recorded values on maximum and total generated power output at a voltage applied to the planar surfaces of the PTC-material of 24 V are given. By maximum generated power output is here understood the output generated at the beginning of the treatment when the circulating liquid has a temperature of about 37°C. The power output will then, in view of the self-regulating capacity of the PTC-material, decrease when approaching the desired treatment temperature as described above. However, it is generally a fact that the higher the maximum output of the central body is, the higher is the output it can generate at a certain treatment temperature provided that sufficient circulation of liquid can be maintained.

From Table 1 it can be seen that a maximum output of about 60 W will be generated by Type 1 at an outer diameter of 7 mm and a length of 30 mm, whereas for Type 2 the same effect will be obtained at a diameter of 6 mm and for Type 3 the diameter of about 5 mm. The alternative to be chosen is dependent on other factors, such as cost of manufacture, suitability for mass production etc. However, it has been found that all three alternatives give fully satisfactory treatment results.

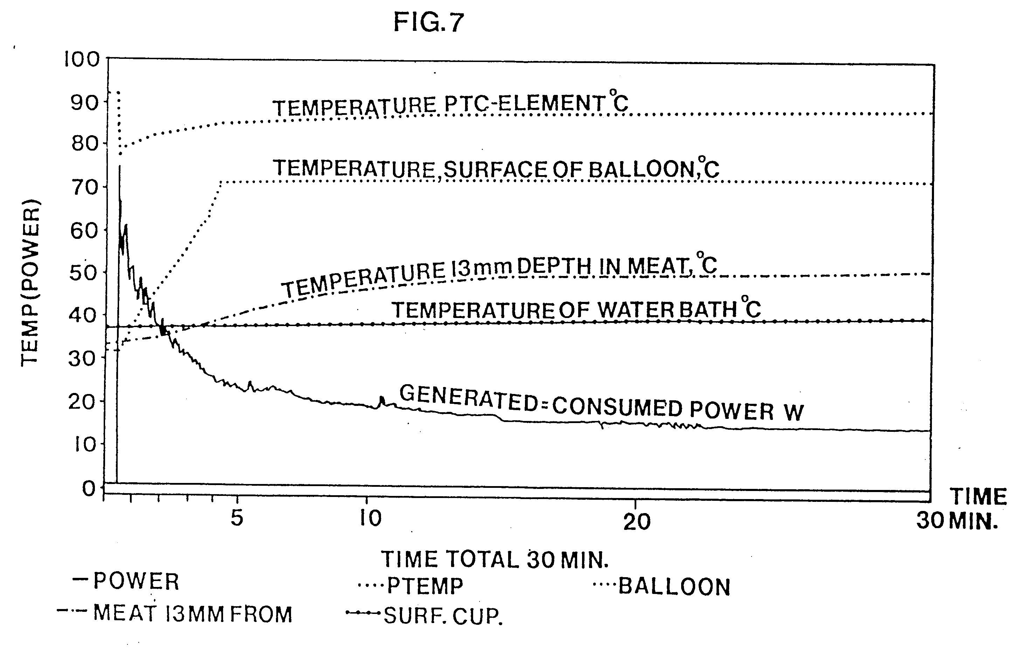

Fig. 7 shows the results of in vitro experiments carried out on meat of beef. The experiments were performed by covering the inside of a thin-walled vessel having approximately the shape of a uterus on the inside with a layer of beef meat in a thickness of about 20-25 mm. The vessel was then placed in a water-bath having a temperature of 37°C. A catheter or instrument corresponding to that described in connection with Fig. 1 and with a central tube or body according to Type 1 of outer diameter 7 mm, generating 60 W as a start up maximum was inserted into the cavity within the meat, and the balloon was then expanded with a liquid consisting of an oxygen-free physiologic sodium chloride solution to a volume of 20 ml. A temperature sensor was place at a distance of about 13 mm within the meat counted from the wall of the expanding balloon. The voltage was then applied and the continued course of the experiment can easily be understood from the diagram shown in Fig. 7.

At the outset the temperature of the PTC-material immediately rose to above 90°C without any generation of power. After half a minute the circulation pump was started and the power output immediately rose to 60-70 W at the same time as the temperature of the PTC-element decreased to 78°C (Curie point 80°C). Already after about 4 minutes the temperature at the surface of the balloon had increased to somewhat above 70°C (treatment temperature), and the energy output had fallen to about 25 W. When continuing the experiment the temperature of the balloon surface remained constant, whereas the temperature 13 mm inside the meat slowly rose from 34°C to about 50°C after about 20 minutes, at the same time as the power output decreased to about 18 W. After about 5 minutes treatment conditions were reached. The treatment is then continued for say 30 to 40 minutes to give the decree result.

A voltage of 24 V was used in the experiments, but even lower voltages can be used in certain cases. Under all circumstances such low voltages are safe. In order to completely eliminate the risk for electro shocks the source of current is suitably for example a rechargeable battery.

As indicated the heat-emission from the PTC-elements is of decisive importance in order to obtain the highest possible power output, which will be obtained by an effective circulation of liquid which also has for a purpose to transfer the absorbed heat to the surface of the balloon and the surrounding tissue.

**TABLE 1**

| | Type 1 | | | Type 2 | | | Type 3 | | | |
|---|---|---|---|---|---|---|---|---|---|---|
| Outer diameter (mm) | 7 | 6 | 5 | 7 | 6 | 5 | 7 | 6 | 5 | 4 |
| Innner diameter (mm) | 6 | 5 | 4 | 6 | 5 | 4 | 5 | 4 | 3 | 2 |
| Power per cm length,W | 20 | 16 | 9.5 | 27 | 21 | 14 | 31 | 25 | 19 | 12,7 |

## Claims

1. A device for carrying out hyperthermia in a body cavity or duct, the access path of which is narrow, comprising an elongate distal section (3) intended to be inserted into said cavity or duct comprising a centrally located, heat-releasing element (19), which is surrounded by an elongate housing, a flexible and/or elastic enclosure (37) surrounding said housing in a liquid-tight manner, further including means (13,15) for supplying energy to the heat-releasing element (19), an axially operating inlet passage (35) at the proximal part of the housing, an outlet (33;68;87) from the housing being arranged for the supply of heat-transmitting medium under pressure for expansion of the flexible enclosure (37) to accomodate to and to exert a controlled pressure on surrounding walls of said cavity or duct, a second inlet to the housing, and means for internal circulation of said medium through the housing, characterized in that the heat-releasing element is self-regulating and comprises a PTC-semi-conductor material having a Curie temperature or Trip point, said heat-releasing element contains two or several axially and parallelly arranged PTC-part-elements having substantially parallel surfaces, said part-elements being separated to form passages between and about the part-elements for effective and even heat-release to the surrounding flow of heat-transmitting medium.

2. A device according to claim 1, characterized in that said semi-conductive material is designed with a packing degree lying within the range about 35 to about 70% by volume.

3. A device according to claim 1 or 2 for the treatment of disorders of the uterus, characterized in that said semi-conductive material has a Trip or Curie point lying within the range about 60°C to about 90°C, particularly about 70°C to about 80°C.

4. A device according to any preceding claim for the treatment of prostatic disorders, characterized in that said semi-conductive material has a Trip or Curie point lying within the range about 45°C to about 65°C, particularly about 48°C to about 56°C.

5. A device according to any preceding claim, characterized in that the thickness of the part-elements is at most about 1.5 mm and that they have an energy generation at steady state of at least about 1 W/cm² exposed element surface at a voltage of about 24 V.

6. A device according to any preceding claim, characterized in that said housing (29) at an outer diameter of about 7-8 mm has the capacity to release at least about 3 W/cm axial length of the housing at the treating temperature involved and at a voltage of about 24 V.

7. A device according to any preceding claim, wherein said means for internal circulation has a capacity at steady state conditions of replacing the void volume of the housing of said medium at least about 30 times per minute.

8. A device according to any preceding claim, characterized by an inlet (31;67;85) for the medium located in the housing on the opposite side of element (19) relative to said outlet (33;68;87), and means for imparting flow to the medium in an internal circuit from the space between housing (29) and the surrounding flexible enclosure (37), through housing (29) via said inlet and outlet for absorbing heat and through the space between housing (29) and enclosure (30) for the release of heat.

9. A device according to claim 8, characterized by at least one first back valve arranged in association with the inlet (31) allowing flow into the interior of housing (29) and the flow resistance of which in an open position is lower than the flow resistance through housing (29), said means (23;25;27) for the flow of the medium in an internal circuit being arranged to provide a reciprocating movement of a small quantity of the pressurized medium enclosed in the inlet canal of the device after expansion of the enclosure, whereby the inlet is closed and the outlet is open, thereby providing circulation of the medium in a closed circuit.

10. A device according to claim 8, characterized by at least one first back valve arranged in association with the outlet (33) allowing flow out of the interior of housing (29), the flow resistance of the inlet (31) being higher than the flow resistance through housing (29), said means (23,25,27) for the flow of the medium in an internal circuit being arranged to provide a reciprocating movement of a small quantity of the pressurized medium enclosed in the inlet canal of the device after expansion of the enclosure, whereby the inlet is open and the outlet is closed, thereby providing circulation of the medium in a closed circuit.

11. A device according to any of claims 1 to 7, characterized by an inlet (31;67;85) for the medium arranged in housing (29) on the other side of element (19) relative to said outlet, and by a partition (43) placed between said inlet (31) and element (19) or between said element (19) and outlet (33), respectively, forming a chamber (32;39) and being provided with an axial aperture containing a second back valve (47) which is oppositely acting relative to a first back valve (49) placed in inlet (31), said means (23,25,27) for the flow of the medium in an internal circuit being arranged to provide a reciprocating movement of a small quantity of the pressurized medium enclosed in the inlet canal of the device after expansion of the enclosure, whereby the inlet is closed and the outlet is open, or the inlet is open and the outlet is closed, respectively, thereby providing circulation of the medium in a closed circuit.

12. A device according to any of claims 9 to 11, characterized in that said means for the flow of the medium are arranged to provide a volumetric flow of the medium, such as by comprising a reciprocating piston.

13. A device according to claim 8, characterized by oppositely operating back valves (49;93, 47;95) arranged in association with inlet (31;85) and outlet (33;87).

14. A device according to claim 11, characterized in that said partition (43) is located between inlet (31) and element (19).

15. A device according to claim 8, characterized in that said means for the flow of the medium are placed between inlet (67) and outlet (69) and arranged to provide axial flow of the medium in the housing.

16. A device according to claim 15, characterized in that said means for the flow of the medium comprise a propeller or an axial pump wheel (39).

17. A device according to claim 15, characterized in that said means for the flow of the medium comprise a reciprocating piston provided with one or several axially directed apertures and corresponding back valves, whereby axial flow of the medium in said housing is provided.

18. A device according to any of claims 1 to 7, 11 and 14 to 17, characterized in that said back valves are of the flap valve type.

## Patentansprüche

1. Gerät zum Durchführen einer Hyperthermie in einer Körperhöhle oder einem Körperkanal, dessen Zugangsweg eng ist, mit einem langgestreckten, distalen Abschnitt (3), der zum Einsetzen in die Höhle oder den Kanal ausgebildet ist, mit einem zentral angeordneten Wärmeabgabeelement (19), das durch ein langgestrecktes Gehäuse umgeben ist, einer flexiblen und/oder elastischen Hülle (37), die das Gehäuse in einer flüssigkeitsdichten Weise umgibt, ferner enthaltend eine Einrichtung (13, 15) zum Zuführen von Energie zum Wärmeabgabeelement (19), einen axial wirkenden Betriebseinlaßdurchtritt (35) am proximalen Teil des Gehäuses, einen Auslaß (36; 68; 87) vom Gehäuse, der für die Zufuhr von Wärmeübertragungsmedium unter Druck für die Expansion der flexiblen Hülle (37) angeordnet ist, um sich an die umgebenden Wände des Hohlraums oder Kanals anzupassen und darauf einen kontrollierten Druck auszuüben, einem zweiten Einlaß zum Gehäuse und eine Einrichtung für eine innere Zirkulation des Mediums durch das Gehäuse**, dadurch gekennzeichnet,** daß das Wärmeabgabeelement selbstregulierend ist und ein PTC-Halbleitermaterial mit einer Curie-Temperatur oder einem Erregungspunkt umfaßt, wobei das Wärmeabgabeelement zwei oder mehrere axial und parallel angeordnete PTC-Teilelemente mit im wesentlichen parallelen Oberflächen enthält, wobei die Teilelemente voneinander getrennt sind, um Durchtritte zwischen und um die Teilelemente zu bilden für eine effektive und gleichmäßige Wärmeabgabe auf die umgebende Strömung des Wärmeübertragungsmediums.

2. Gerät nach Anspruch 1, **dadurch gekennzeichnet,** daß das Halbleitermaterial mit einer Packungsdichte ausgebildet ist, die innerhalb des Bereichs von etwa 35 bis etwa 70 Vol-% liegt.

3. Gerät nach Anspruch 1 oder 2 für die Behandlung von Störungen des Uterus, **dadurch gekennzeichnet,** daß das Halbleitermaterial einen Erregungs- oder Curiepunkt aufweist, der innerhalb des Bereichs von etwa 60°C bis etwa 90°C, insbesondere von etwa 70°C bis etwa 80°C liegt.

4. Gerät nach irgendeinem vorangegangenen Anspruch für die Behandlung von Prostatastörungen, **dadurch gekennzeichnet,** daß das Halbleitermaterial einen Erregungsoder Curiepunkt aufweist, der innerhalb des Bereichs von etwa 45°C bis etwa 65°C, insbesondere etwa 48°C bis etwa 56°C liegt.

5. Gerät nach irgendeinem vorangegangenen Anspruch, **dadurch gekennzeichnet,** daß die Dicke der Teilelemente höchstens etwa 1,5 mm aufweist, und daß sie eine Energieerzeugung beim stabilen Zustand von mindestens etwa 1 W/cm² der freiliegenden Elementoberfläche bei einer Spannung von etwa 24 V aufweisen.

6. Gerät nach irgendeinem vorangegangenen Anspruch, **dadurch gekennzeichnet,** daß das Gehäuse (29) an einem äußeren Durchmesser von etwa 7 bis 8 mm die Kapazität aufweist, mindestens etwa 3 W/cm der axialen Länge des Gehäuses bei der entsprechenden Behandlungstemperatur und bei einer Spannung von etwa 24 V abzugeben.

7. Gerät nach irgendeinem vorangegangenen Anspruch, wobei die Einrichtung für die innere Zirkulation eine Kapazität im stabilen Zustand aufweist, um das Leervolumen des Gehäuses des Mediums mindestens 30 x pro Minute zu ersetzen.

8. Gerät nach irgendeinem vorangegangenen Anspruch, **gekennzeichnet durch** einen Einlaß (31; 67; 85) für das Medium, der im Gehäuse an der gegenüberliegenden Seite des Elementes (19) bezüglich des Ausgangs (33; 68; 87) angeordnet ist, und eine Einrichtung zum Erzeugen einer Strömung des Mediums in einem inneren Kreislauf vom Zwischenraum zwischen dem Gehäuse (29) und der umgebenden, flexiblen Hülle (30), durch das Gehäuse (29) über den Einlaß und den Auslaß zum Absorbieren von Wärme und durch den Zwischenraum zwischen dem Gehäuse (29) und der Hülle (30) für die Abgabe der Wärme.

9. Gerät nach Anspruch 8, **gekennzeichnet durch** mindestens ein erstes Rückschlagventil, das in Zusammenwirkung mit dem Einlaß (31) angeordnet ist, und eine Strömung in das Innere des Gehäuses (29) gestattet, wobei sein Strömungswiderstand in Offenstellung geringer als der Strömungswiderstand durch das Gehäuse (29) ist, wobei die Einrichtung (23; 25; 27) für die Strömung des Mediums in einem inneren Kreislauf so angeordnet ist, um eine hin- und hergehende Bewegung einer kleinen Menge des im Einlaßkanal des Gerätes nach der Expansion der Hülle eingeschlossenen Druckmediums zu erzeugen, wodurch der Einlaß geschlossen und der Auslaß offen ist, wobei dadurch eine Zirkulation des Mediums in einem geschlossenen Kreislauf erzeugt wird.

10. Gerät nach Anspruch 8, **gekennzeichnet durch** mindestens ein erstes Rückschlagventil, das in Zusammenwirkung mit dem Auslaß (33) angeordnet ist, der die Strömung aus dem Inneren des Gehäuses (29) heraus gestattet, wobei der Strömungswiderstand des Einlasses (31) höher als der Strömungswiderstand durch das Gehäuse (29) ist, wobei die Einrichtung (23, 25, 27) für die Strömung des Mediums in einem inneren Kreislauf so angeordnet ist, daß eine hin- und hergehende Bewegung einer im Einlaßkanal des Gerätes nach der Expansion der Hülle eingeschlossenen, kleinen Menge des Druckmediums verursacht wird, wodurch der Einlaß offen und der Auslaß geschlossen ist, wobei dadurch eine Zirkulation des Mediums in einem geschlossenen Kreislauf bewirkt wird.

11. Gerät nach irgendeinem der Ansprüche 1 bis 7, **gekennzeichnet durch** einen Einlaß (31; 67; 85) für das Medium, der im Gehäuse (29) an der anderen Seite des Elementes (19) relativ zum Auslaß angeordnet ist, und durch eine Trennung (43), die jeweils zwischen dem Einlaß (31) und dem Element (19) oder zwischen dem Element (19) und dem Auslaß (33) angeordnet ist, die eine Kammer (32; 39) bildet und mit einer axialen Öffnung versehen ist, die ein zweites Rückschlagventil (47) enthält, das bezüglich eines ersten Rückschlagventils (49), das im Einlaß (31) angeordnet ist, entgegengesetzt wirkt, wobei die Einrichtung (23, 25, 27) für die Strömung des Mediums in einem inneren Kreislauf so angeordnet ist, daß eine hin- und hergehende Bewegung einer im Einlaßkanal des Gerätes nach der Expansion der Hülle eingeschlossenen, kleinen Menge des Druckmediums verursacht wird, wodurch jeweils der Einlaß geschlossen und der Auslaß offen oder der Einlaß offen und der Auslaß geschlossen ist, wobei dadurch eine Zirkulation des Mediums in einem geschlossenen Kreislauf bewirkt wird.

12. Gerät nach einem der Ansprüche 9 bis 11**, dadurch gekennzeichnet,** daß die Einrichtung für die Strömung des Mediums so angeordnet ist, daß eine volumetrische Strömung des Mediums bewirkt wird, beispielsweise durch die Anordnung eines hin- und hergehenden Kolbens.

13. Gerät nach Anspruch 8, **gekennzeichnet durch** entgegengesetzt wirkende Rückschlagventile (49; 93, 47; 95), die in Zusammenwirkung mit dem Einlaß (31; 85) und dem Auslaß (33; 87) angeordnet sind.

14. Gerät nach Anspruch 11, **dadurch gekennzeichnet,** daß die Trennung (43) zwischen dem Einlaß (31) und dem Element (19) angeordnet ist.

15. Gerät nach Anspruch 8, **dadurch gekennzeichnet,** daß die Einrichtung für die Strömung des Mediums zwischen dem Einlaß (67) und dem Auslaß (69) angeordnet und so ausgebildet ist, daß eine axiale Strömung des Mediums im Gehäuse erzeugt wird.

16. Gerät nach Anspruch 15, **dadurch gekennzeichnet,** daß die Einrichtung für die Strömung des Mediums einen Propeller oder ein axiales Pumpenrad (39) umfaßt.

17. Gerät nach Anspruch 15, **dadurch gekennzeichnet,** daß die Einrichtung für die Strömung des Mediums einen hin- und hergehenden Kolben umfaßt, der mit einem oder mehreren, axial ausgerichteten Öffnungen und korrespondierenden Rückschlagventilen versehen ist, wodurch die axiale Strömung des Mediums im Gehäuse erzeugt wird.

18. Gerät nach irgendeinem irgendeinem der Ansprüche 1 bis 7, 11 und 14 bis 17, **dadurch gekennzeichnet,** daß die Rückschlagventile in der Art von Klappenventilen ausgebildet sind.

## Revendications

1. Dispositif pour réaliser une hyperthermie dans une cavité ou un canal corporel, dont le chemin d'accès est étroit, comprenant une section distale allongée (3) destinée à être insérée dans ledit canal ou cavité comprenant un élément libérant de la chaleur et situé au centre (19), qui est entouré par un boîtier allongé, une enceinte flexible et/ou élastique (37) entourant ledit boîtier d'une manieré étanche aux liquides, comprenant en outre des moyens (13, 15) pour délivrer de l'énergie à l'élément libérant de la chaleur (19), un passage d'entrée (35) fonctionnant de façon axiale au niveau de la partie proximale du boîtier, une sortie (33 ; 68 ; 87) à partir du boîtier étant agencée pour la délivrance d'un milieu transmetteur de chaleur sous pression pour la dilatation de l'enceinte flexible (37) afin qu'elle s'adapte A et exerce une pression contrôlée sur des parois environnantes dudit canal ou cavité, une deuxième entrée vers le bôitier, et des moyens pour la circulation interne dudit milieu au travers du boîtier, caractérisé en ce que l'élément libérant de la chaleur est à auto-régulation et comprend un matérieau semi-conducteur en PTC ayant un certain point de Curie ou de déclenchement, ledit élément libérant de la chaleur contient deux ou plus de deux éléments partiels en PTC agencés de façon axiale et parallèle, ayant des surfaces pratiquement parallèles, lesdits éléments partiels étant séparés pour former des passages entre et autour des éléments partiels pour une libération de chalcur efficace et uniforme vers l'écoulement environnant de milieu transmetteur de chaleur.

2. Dispositif selon la revendication 1, caractérisé en ce que ledit matériau semi-conducteur est conçu avec un degé de tassement situé dans la plage allant d'environ 35 à environ 70 % en volume.

3. Dispositif selon la revendication 1 ou 2 pour le traitement de troubles de l'utérus, caractérisé en ce que ledit matériau semi-conducteur a un point de déclenchement ou de Curie situé dans la plage allant d'environ 60°C à environ 90°C, en particulier d'environ 70°C à environ 80°C.

4. Dispositif selon l'une quelconque des revendications précédentes pour le traitement de troubles prostatiques, caractérisé en ce que ledit matériau semi-conducteur a un point de déclenchement ou de Curie situé dans la plage allant d'environ 45°C à environ 65°C, en particulier d'environ 48°C à environ 56°C.

5. Dispositif selon l'une quelconque des revendications précédentes, caractérisé en ce que l'épaisseur des éléments partiels est au plus d'environ 1,5 mm et en ce qu'ils présentent une génération d'énergie à l'état stable d'au moins environ 1 W par cm² de surface d'élément exposée, sous une tension d'environ 24 V.

6. Dispositif selon l'une quelconque des revendications précédentes, caractérisé en ce que ledit boîtier (29) ayant un diamètre extérieur d'environ 7 à 8 mm a la capacité de libérer au moins 3 W par cm de longueur axiale du boîtier à la température de traitement mise en jeu et sous une tension d'environ 24 V.

7. Dispositif selon l'une quelconque des revendications précédentes, dans lequel lesdits moyens pour la circulation interne ont une capacité, dans des conditions d'état stable, de remplacement du volume de vide du boîtier dudit milieu d'au moins environ 30 fois par minute.

8. Dispositif selon l'une quelconque des revendications précédentes, caractérisé par une entrée (31 ; 67 ; 85) pour le milieu situé dans le boîtier sur le côté opposé de l'élément (19) par rapport à ladite sortie (33 ; 68 ; 87), et des moyens pour conférer un écoulement au milieu dans un circuit interne depuis l'espace entre le boîtier (29) et l'enceinte flexible environnante (37), au travers du boîtier (29) par l'intermédiaire desdites entrée et sortie pour absorber de la chaleur et au travers de l'espace entre le boîtier (29) et l'enceinte (30) pour la libération de chaleur.

9. Dispositif selon la revendication 8, caractérisé par au moins une première vanne de retour agencée en association avec l'entrée (31) permettant un écoulement dans l'intérieur du boîtier (29) et dont la résistance à l'écoulement dans une position ouverte est inférieure à la résistance à l'écoulement au travers du boîtier (29), lesdits moyens (23 ; 25 ; 27) pour l'écoulement du milieu dans un circuit interne étant agencés de façon à réaliser un mouvement de va-et-vient d'une petite quantité du milieu pressurisé enfermé dans le canal d'entrée du dispositif après dilatation de l'enceinte, grâce à quoi l'entrée est fermée et la sortie est ouverte, réalisant ainsi la circulation du milieu dans un circuit fermé.

10. Dispositif selon la revendication 8, caractérisé par au moins une première vanne de retour agencée en association avec la sortie (33) permettant un écoulement de l'intérieur du boîtier (29), la résistance à l'écoulement de l'entrée (31) étant supérieure à la résistance à l'écoulement au travers du boîtier (29), lesdits moyens (23, 25, 27) pour l'écoulement du milieu dans un circuit interne étant agencés de façon à réaliser un mouvement de va-et-vient d'une petite quantité du milieu pressurisé enfermé dans le canal d'entrée du dispositif après dilatation de l'enceinte, grâce à quoi l'entrée est ouverte et la sortie est fermée, réalisant ainsi la circulation du milieu dans un circuit fermé.

11. Dispositif selon l'une quelconque des revendications 1 à 7, caractérisé par une entrée (31 ; 67 ; 85) pour le milieu agencée dans le boîtier (29) de l'autre côté de l'élément (19) par rapport à ladite sortie, et par une séparation (43) placée entre ladite entrée (31) et l'élément (19) ou entre ledit élément (19) et la sortie (33), respectivement, formant une chambre (32 ; 39) et étant munie d'une ouverture axiale contenant une deuxième vanne de retour (47) qui agit à l'opposé par rapport à une première vanne de retour (49) placée dans l'entrée (31), lesdits moyens (23, 25, 27) pour l'écoulement du milieu dans un circuit interne étant agencés de façon à réaliser un mouvement de va-et-vient d'une petite quantité du milieu pressurisé enfermé dans le canal d'entrée du dispositif après dilatation de l'enceinte, grâce à quoi l'entrée est fermée et la sortie est ouverte, ou bien l'entrée est ouverte et la sortie est fermée, respectivement, réalisant ainsi la circulation du milieu dans un circuit fermé.

12. Dispositif selon l'une quelconque des revendications 9 à 11, caractérisé en ce que lesdits moyens pour l'écoulement du milieu sont agencés de façon à permettre un écoulement volumétrique du milieu, par exemple en comprenant un piston alternatif.

13. Dispositif selon la revendication 8, caractérisé par des vannes de retour (49 ; 93, 47 ; 95) à fonctionnement opposé, agencées en association avec l'entrée (31 ; 85) et la sortie (33 ; 87).

14. Dispositif selon la revendication 11, caractérisé en ce que ladite séparation (43) est située entre l'entrée (31) et l'élément (19).

15. Dispositif selon la revendication 8, caractérisé en ce que lesdits moyens pour l'écoulement du milieu sont placés entre l'entrée (67) et la sortie (69) et agencés de façon à permettre un écoulement axial du milieu dans le boîtier.

16. Dispositif selon la revendication 15, caractérisé en ce que lesdits moyens pour l'écoulement du milieu comprennent un propulseur ou une roue mobile de pompe axiale (39).

17. Dispositif selon la revendication 15, caractérisé en ce que lesdits moyens pour l'écoulement du milieu comprennent un piston alternatif muni d'une ou plusieurs ouvertures dirigées de façon axiale et vannes de retour correspondantes, grâce à quoi l'écoulement axial du milieu dans ledit boîtier est réalise.

18. Dispositif selon l'une quelconque des revendications 1 à 7, 11 et 14 à 17, caractérisé en ce que lesdites vannes de retour sont du type à vanne papillon.
